# EUROPEAN PATENT SPECIFICATION

(11) **EP 2 808 042 B1**
(45) Date of publication and mention of the grant of the patent: **23.03.2016**
(21) Application number: 14170433.8
(22) Date of filing: 28.05.2014
(51) Int. Cl.: A61M 1/16

(54) **Bag containing bicarbonate powder**
Beutel mit Bicarbonatpulver
Sac contenant une poudre de bicarbonate

(30) Priority: 28.05.2013 IT BO20130266
(43) Date of publication of application: 03.12.2014
(73) Proprietor: Bellco S.r.l., Mirandola (IT)
(72) Inventor: Scala, Maurizio, 98026 Nizza di Sicilia (IT); Puviani, Fabrizio, 41038 San Felice Sul Panaro (IT); Lupotti, Marco, 41030 Sorbara di Bomporto (IT); Tortola, Raffaello, 86080 Miranda (IT)
(74) Representative: Boggio, Luigi

(56) References cited:
- EP-A2- 2 025 355
- WO-A2-2010/017906
- DE-A1-102011 017 048
- DE-U1- 20 200 689
- US-A- 5 385 564

## Description

The present invention relates to a bag containing bicarbonate powder, in particular a bag containing sodium bicarbonate powder, which can be connected to a dialysis machine so as to produce a saturated bicarbonate solution.

Containers are known that hold sodium bicarbonate powder and can be connected to the hydraulic circuit of the dialysis machine supplying a sodium bicarbonate solution to the dialysis filter so as to receive, from the hydraulic circuit, a solvent, typically water, and to supply, to the hydraulic circuit, a saturated bicarbonate solution produced by the dissolution of the bicarbonate powder in the solvent flowing through the container. These containers are disposable medical devices that, after having been used, are classified as special waste, namely waste that requires a specific disposing procedure, whose costs are substantially proportional to the volume of the material to be disposed of.

A type of container is known that holds bicarbonate powder and is made of a flexible plastic material in order to reduce manufacturing and transport costs and in order to ensure a limited size after the use. This flexible container basically consists of a plastic film bag containing bicarbonate powder and closed in a tight manner by a fitting provided with an inlet for the solvent and an outlet for the saturated bicarbonate solution. The container comprises, furthermore, a dip tube consisting of a straw, which, with one end, is connected to the outlet of the fitting and, with the other end, is dipped to the bottom of the bag so as to draw out the saturated solution without sucking in air, and a filter, which is connected to the dipped end of the straw so as to prevent bicarbonate powder from entering the hydraulic circuit of the dialysis machine. The fitting is provided with an additional mouth having a section that is larger than said inlet and outlet, so as to allow the bag to be filled with bicarbonate powder after the container has been assembled. After the filling with bicarbonate powder has ended, the additional mouth is closed.

The flexible container described above, when it is empty, after having been used, always takes up a significant space, even if it has been flattened, due to the presence of the straw and of the filter and due to the large size of the additional mouth. Furthermore, the presence of the straw and of the filter and the need to close the additional mouth after the bag has been filled are a possible source of complications for the container manufacturing process, which keep the manufacturing costs high.

Finally, despite the presence of the dip tube, a certain quantity of gas always enters the hydraulic circuit of the dialysis machine, this gas basically consisting of the carbon dioxide produced by the chemical reaction between the solvent and the bicarbonate powder and of the air contained in the particles of the bicarbonate powder. This gas is strongly undesired because dialysis liquid cannot contain gas and because the presence of gas bubbles in the hydraulic circuit can negatively affect the dynamics control function performed by the servosystems of the dialysis machine.

European patent application EP2025355A2 discloses a container for dialysis purposes, which comprises a water inlet and a solution outlet which are located on the same side of the container, preferably within its bottom zone.

German utility model DE20200689U1 discloses a welding boat for a pouch or bag having a center part and two prolongations extending, starting from this, in opposite directions and tapering acutely, which is provided, on the one hand, with an opening for the filling of the pouch with a substance in manufacture and, on the other hand, with inlets and outlets for the actual use of the pouch. The center part is substantially filled by an opening with a lumen which is as large as possible and preferably circular and the side edges of the side prolongations contact the opening tangentially.

German patent application DE102011017048A1 discloses a multichamber container and a method for preparing medical fluids composed of concentrates which make a contribution toward the electrical conductivity of the solution and are additionally prepared from concentrates which do not make a contribution toward the electrical conductivity of the solution. The multichamber container is constructed by dividing the solution components into multiple chambers, so that following the dissolving due to breaking open of the first chamber border of a solution component, which makes a contribution toward the electrical conductivity of the medicinal solution, another chamber border of another solution component which does not make any contribution toward the electrical conductivity of the solution is broken open.

International application WO2010/017906A2 discloses a connector for a replaceable container to be used in a medical machine, which is comprised by at least a body, the inner side of which is traversed by inlet and outlet tubes extending from within such container towards an outer side of the connector, wherein said tubes separately extend leading to two laterally spaced-apart apertures that are removably attachable to a mounting means and supplementary connection of said machine.

U.S. patent US5385564 discloses a system for packaging and using granulated solid dialysate concentrate. A bag is constructed to hold granulated or powder concentrate, having a V-shaped bottom with an access port in the bottom. Water is flowed through the access port and into the bag using suitable water control means. The incoming water lifts and suspends the granules or powder in a turbulent flow, thereby filling the container simultaneous with dissolving the granules or powder. The water with dissolve concentrate then constitutes the dialysis solution and is ready for use or transfer to a dialysate delivery machine.

The object of the present invention is to provide a bag containing bicarbonate powder, which is designed to eliminate the aforementioned drawbacks and, at the same time, can be manufactured in a straightforward and low-cost manner.

The present invention provides a bag containing bicarbonate powder according to the appended claims.

The present invention will now be described with reference to the accompanying drawings, which show a nonlimiting embodiment thereof, wherein:
- figure 1 shows, in a plan view, the bag containing bicarbonate powder according to the present invention;
- figure 2 shows, in a perspective view, the bag of figure 1;
- figure 3 shows the lower part of the bag of figure 1, when the lower part is folded;
- figure 4 shows the flow chart of a method to manufacture the bag of figure 1;
- figure 5 shows a semifinished product obtained at an intermediate stage of the manufacturing method of figure 4;
- figure 6 shows, in a perspective view, a coupling assembly of a dialysis machine for the coupling of the connection of the bag of figure 1 to a hydraulic circuit of the machine for the distribution of a solvent and of a bicarbonate solution produced with said solvent;
- figures from 7 to 10 show, in a perspective view and partially in section, the coupling assembly of figure 6 in different operating configurations; and
- figure 11 shown, in a schematic manner, the hydraulic circuit of the dialysis machine and part of the coupling assembly of figure 6, which connects the bag of figure 1 to the hydraulic circuit.

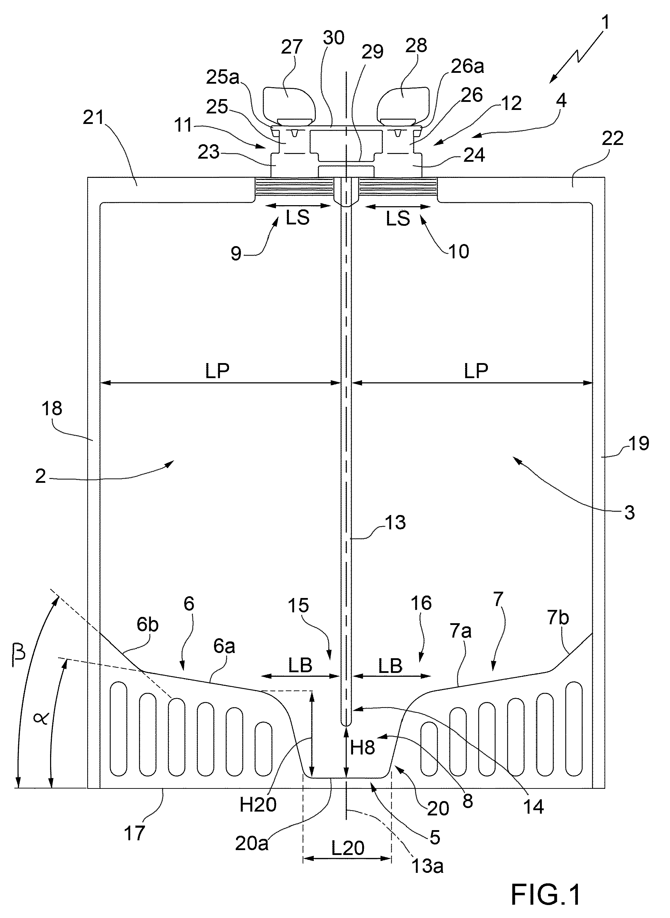

In figure 1, the bag according to the present invention is indicated with number 1 and is shown empty and flattened on the plane of the plan view. The bag 1 is divided into two pockets 2 and 3, which are suited to contain bicarbonate powder, and comprises a lower U-shaped channel 5, which establishes a communication between the bottom portions, hereinafter simply referred to as bottoms 6 and 7, of the two pockets and has a central section or portion 8 that is completely arranged under the minimum level of both bottoms 6 and 7, so that the lower channel 5 is normally full of bicarbonate powder, when, in use, the bag 1 is held with the bottoms 6 and 7 facing downwards. The pockets 2 and 3 and the lower channel 5 are manufactured as one single piece from a plastic film that is folded in two parts and welded. The plastic film is, for example, a polyamide-polyethylene (PA-PE) film. Each pocket 2, 3 is closed on the upper side, except for a respective upper opening 9, 10. The bag 1 comprises, furthermore, an upper connection 4 for the connection to the hydraulic circuit of a dialysis machine. The upper connection 4 comprises two passage elements 11 and 12, each of which is welded in a tight manner to the upper opening 9 and 10 of a respective pocket 2, 3, so as to allow a solvent, consisting for example of water, to be introduced into the bag (1) through one of the passage elements, for example element 11, and a saturated bicarbonate solution to flow out through the other passage element (12), after the solvent has spread through the bicarbonate powder from the pocket 2 to the pocket 3 flowing through the lower channel 5. The bag 1 lacks any kind of dip tube and relative filter.

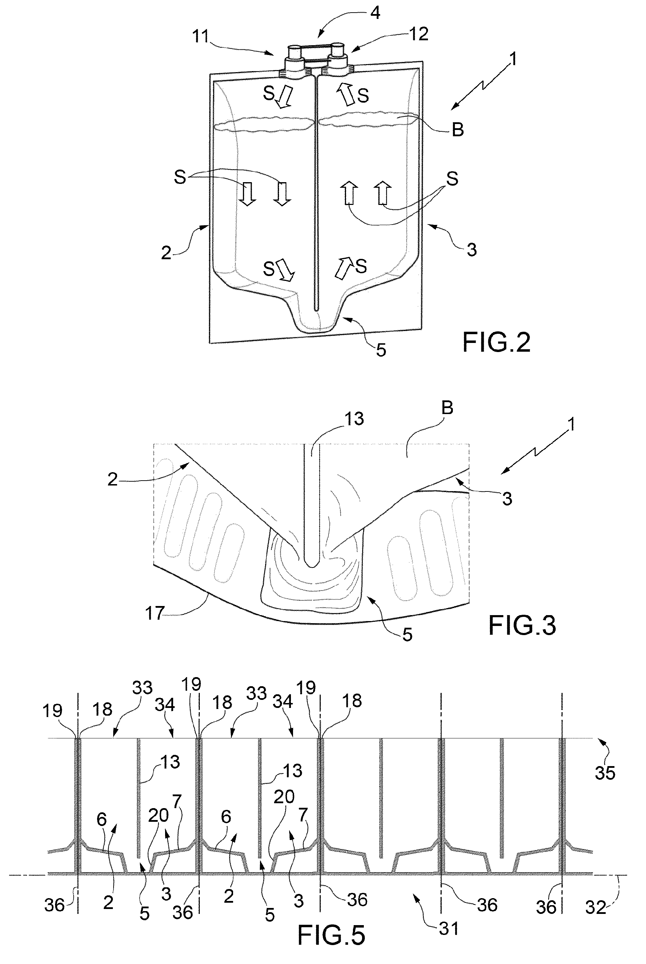

Figure 2 shows the bag 1 filled with sodium bicarbonate powder, indicated with B. The arrows indicated with S in figure 2 show the flow of the solvent, which flows in through the passage element 11, flows from the pocket 2 to the pocket 3, flows through the lower channel 5, and flows out of the passage element 12. The solvent consists, for example, of water. As the solvent flows in through the passage element 11, the saturated bicarbonate solution flows out of the other passage element 12.

With reference to figure 1, again, the lower channel 5 is delimited on the upper side by an inner side 14, which is convex, and on the lower side by an outer side 20, which is substantially concave. The two pockets 2 and 3 are symmetrical relative to a longitudinal symmetry axis 13a of the bag 1 and have the same capacity. In particular, the bag 1 has a central welded joint 13, which extends along the axis 13a so as to divide the bag 1 into the two pockets 2, 3 and so as to define, with an end of its, the inner side 14 of the lower channel 5. The bottoms 6 and 7 of the pockets 2 and 3 have, in symmetrical and close positions relative to the axis 13a, respective lower openings 15 and 16 that are contiguous with said central welded joint 13. The lower channel 5 is symmetrical relative to the axis 13a, as well, and establishes a communication between the lower openings 15 and 16. In other words, each one of the two branches of the lower channel 5 communicates with the lower opening 15, 16 of the respective pocket 2, 3.

The bottoms 6 and 7 are inclined towards the respective lower openings 15 and 16 in a specular manner relative to the axis 13a. In particular, each bottom 6, 7 comprises a first straight portion 6a, 7a, which is joined to the respective lower opening 15, 16 and is inclined, relative to a direction defined by the straight lower edge 17 of the bag 1, with an angle α ranging from 5° to 15°, and a second portion 6b, 7b, which extends from a respective lateral welded joint 18, 19 of the bag 1, which defines the outer side of the respective pocket 2, 3, to the first portion 6a, 7a and is inclined, relative to the lower edge 17, with an angle β ranging from 40° to 50°. As one can assume from figure 1, the lateral welded joints 18 ans 19 are parallel to the welded joint 13 and the lower edge 17 is perpendicular to the axis 13a.

Each lower opening 15, 16 having a width LB, which is measured perpendicular to the axis 13a and in the flattening plane of the bag 1 and is smaller than the half, and in particular smaller than one third, of the inner width LP of a pocket 2, 3, which is measured perpendicular to the axis 13a, as well. The first portion 6a, 7a of each bottom 6, 7 is joined to an end segment of the outer side 20 of the channel 5. The outer side 20 is substantially concave, in that it comprises a straight central segment defining the bottom 20a of the channel. The bottom 20a has a width L20, which is measured perpendicular to the axis 13a and is larger than the width LB, and in particular equal to approximately one third of the width LP. Each one of the two branches of the channel 5 has a length H20, which is measured parallel to the axis 13a between the lowest point of the straight portions 6a and 7a of the bottoms 6 and 7 and the bottom 20a of the channel 5 and is approximately equal to the width L20, in particular equal to approximately one third of the width LP. The central section 8 of the channel has a depth H8, which is measured along the axis 13a and is smaller that two thirds of the length H20.

The bottoms 6 and 7 and the outer side 20 of the channel 5 define a bottom line of the bag 1, which is obtained by welding, according to a corresponding shape, the lower part of the bag 1, namely the part that originates from the folding of the plastic film along the edge 17.

The upper openings 9 and 10 are in symmetrical and close positions relative to the axis 13a and are contiguous with the central welded joint 13. Each pocket 2, 3 comprises two upper flaps, which are partly welded to one another so as to form a respective upper welded joint 21, 22, that is contiguous with a respective lateral welded joint 18, 19, and, for the remaining part, define the respective opening 9, 10. Each upper opening 9, 10 has a width LS, which is measured perpendicular to the axis 13a and is substantially equal to approximately one third of the length LP.

The central welded joint 13 has a width, which is measured perpendicular to the axis 13a and ranges from 3 to 7 mm. The lateral welded joints 18 and 19 have a width that slightly larger than the one of the central welded joint 13, for example ranging from 4 to 8 mm. The upper welded joints 21 and 22 have a width, which is measured parallel to the axis 13a and ranges from 10 to 14 mm.

The passage elements 11 and 12 have a substantially circular symmetry and are identical to one another. Each passage element 11, 12 comprises a respective base portion 23, 24, whose outer peripheral surface is tightly welded to the edges of the respective upper opening 9, 10, and a respective end portion 25, 26, which is closed, on the upper side, by a respective plug 27, 28 that can be removed by hand, by breaking it, so as to permit the connection to a respective branch of a hydraulic circuit (not shown) of a dialysis machine.

The connection 4 comprises, furthermore, two flat elements 29 and 30, which are parallel to one another and substantially perpendicular to the axis 13a, so as to rigidly connect the passage elements 11 and 12 to one another, keeping a given distance. In particular, the flat element 29 connects the base portions 23 and 24, while the other flat element connects the end portions 25 and 26. The end portions 25 and 26 have respective annular edges 25a and 26a, which are coplanar and joined to the flat element 30. The connection 4 is entirely made of an injection-moulded plastic material.

In the example of figure 1, the dimensional parameters of the bag 1 are the following:
- overall height of the bag, measured along the axis 13a, excluding the connection 4, equal to 295 mm;
- overall width of the bag, measured transversely to the axis 13a, equal to 250 mm;
- width LP approximately equal to 116 mm;
- width L20 equal to 42 mm;
- height H20 equal to 42 mm;
- depth H8 equal to 25 mm;
- width LS equal to 38 mm;
- angle α approximately equal to 10°;
- angle β approximately equal to 45°;

The bag 1 of the example of figure 1 is shaped so as to hold 1300 g of sodium bicarbonate powder. For smaller quantities of bicarbonate, the bag 1 is similar to the one of figure 1, with the only difference that it has a smaller overall height.

The shape of the bag 1 is divided into the two pockets 2 and 3, which communicate at the bottom by means of the channel 5, which acts as a communicating vessel, thus eliminating the need for a dip tube and a relative filter. The absence of dip tubes and filters allows manufacturers to produce a bag that, after having been used and flattened, takes up a very small place. Furthermore, thanks to the lack of a dip tube, the number of components making up the bag 1 is significantly reduced: plastic film, bicarbonate powder, and the connection 4 for the coupling to the dialysis machine.

Thanks to the particular configuration described above, the channel 5 never completely closes during the use of the bag 1, since, when the bag 1 filled with bicarbonate powder B folds in correspondence to the channel 5, different folding lines are formed therein, which always leave a free passage between the two pockets 2 and 3, as one can assume from the example of figure 3. Furthermore, the relatively small sizes of the channel 5 allow the residual quantity of bicarbonate left in the bag at the end of a dialysis treatment to be minimized. As a matter of fact, the residual quantity of bicarbonate is substantially the one located in the trap defined by the portion of the channel 5 arranged under the imaginary line defined by the depth H8.

The absence of a dip tube permits another advantage, which is that of further simplifying the process performed to assemble and fill the bag 1.

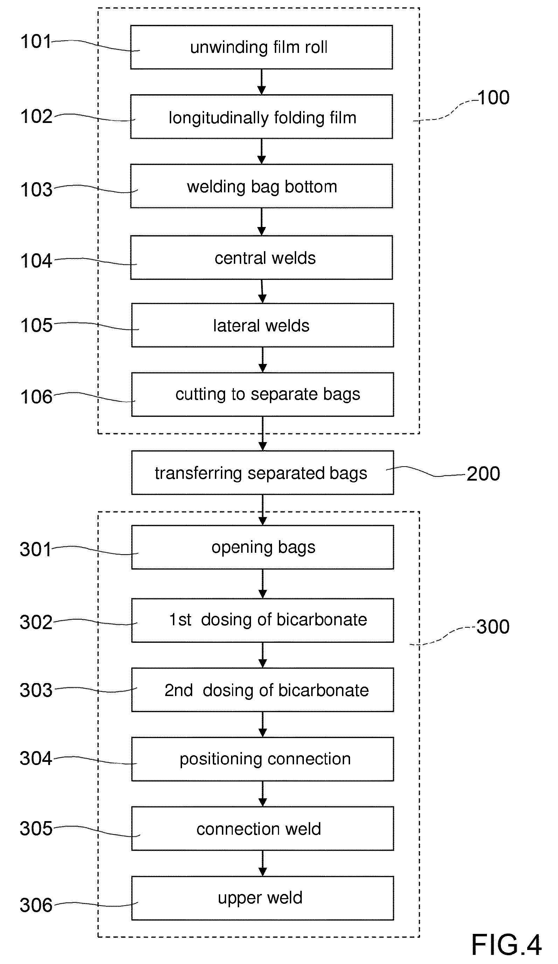

The method used to manufacture the bag 1 is implemented by an automatic apparatus of the FFS type (Form, Fill and Seal) comprising a series of operating stations controlled by a control unit. Figure 4 shows the sequence of the main processing steps performed by the manufacturing apparatus. Each one of the steps shown can be carried out by a respective operating station of the manufacturing apparatus. Figure 5 shows a semifinished product at an intermediate stage of the method to manufacture the bag 1.

With reference to figures 4 and 5, the production of the bag starts by unwinding the plastic film from a roll (step 101). The plastic film is folded along its longitudinal middle line while it is fed (step 102). The folded plastic film is fed discontinuously by means of a system of friction rollers. The feeding pitch of the friction roller system is equal to the overall width of a bag 1.

At this point, the folded film is welded in different parts so as to form a semifinished product 31 (figure 5), which comprises a succession of open bags, each of which consists of a respective pair of pockets (2, 3) and of the relative channel 5 of the bag 1, the pockets 2 and 3 being arranged transverse to the folding line 32 and being partially welded so as to be open in correspondence to respective upper sides 33 and 34, which are aligned along a side 35 of the folded film that is opposite to the side defined by the folding line 32.

In particular, on the side of the folding line 32, a series of welds having a predefined profile are performed in order to form the bottom of the bags (step 103), namely to form the bottom line of each bag 1 defined by the bottoms 6 and 7 of the pockets 2 and 3 and by the outer side 20 of the channel 5. Then, one needs to perform a series of straight welds transverse to the folding line 32, each of which corresponds to the central weld 13 of a respective bag 1 (step 104), and a further series of straight welds transverse to the folding line 32, which are alternated, in the direction of the folding line 32, with the welds 13 and are parallel thereto and each correspond to the joining of the lateral welds 18 and 19 of two bags 1 that are contiguous with one another (105).

Each weld 13, each pair of welds 18 and 19 as well as each shaped weld of the bottom of the bag 1 are performed one at a time in correspondence to relative welding stations. Each welding step is performed by means of a respective group of plates heated by electrical resistors and having the shape of the welds to be performed. Furthermore, each welding step is followed by a respective weld cooling step.

Finally, the semifinished product 31is cut along cutting lines 36 (figure 5) coinciding with the middle lines of the transverse welds 18, 19, so as to separate the open bags from one another (step) 106, namely so as to separate the pairs of pockets 2, 3 from one another in such a way that they are laterally defined by the respective lateral welded joints 18 and 19.

Steps 101-106, which are indicated as a whole with number 100 in figure 4, are carried out in a series of stations arranged in a line and run through by a linear conveying system which feeds, at first, the folded plastic film, then the semifinished product and in the end the separated open bags. The following processing steps, which are indicated as a whole with number 300, are carried out in a series of stations arranged around a carousel conveyor and substantially deal with the filling of the pockets 2 and 3 with the bicarbonate powder and with the closing thereof with the insertion of the relative connection 4 for the connection to the dialysis machine. The separated open bags are transferred, on by one, from the linear conveying system to the carousel conveyor by means of a mechanical hand (step 200).

In particular, after having been placed on the carousel conveyor, the pockets 2 and 3 are opened starting from the upper sides 33 and 34, namely the flaps of the upper side 33, 34 of each pocket 2, 3 are mutually moved apart so as to allow the open bag to be filled (step 301), and then they are simultaneously filled with bicarbonate powder, thus introducing the latter through the upper sides 33 and 34 by means of two subsequent dosing steps (steps 302 and 303). For each open bag that has been filled, the connection 4 is positioned with the respective passage elements 11 and 12 in the two upper sides 33 and 34 (step 304), the connection 4 is welded to part of the flaps of the upper sides 33 and 34 (step 305) and the remaining part of the flaps of the upper sided 33 and 34 is welded so as to completely close the pockets (step 306), namely by forming the welded joints 21 and 22 shown in figure 1, thus obtaining the finished bag 1.

The bag 1 obtained with the manufacturing method described above can be used with a dialysis machine, which, in the figures from 6 to 11, is indicated as a whole with number 37.

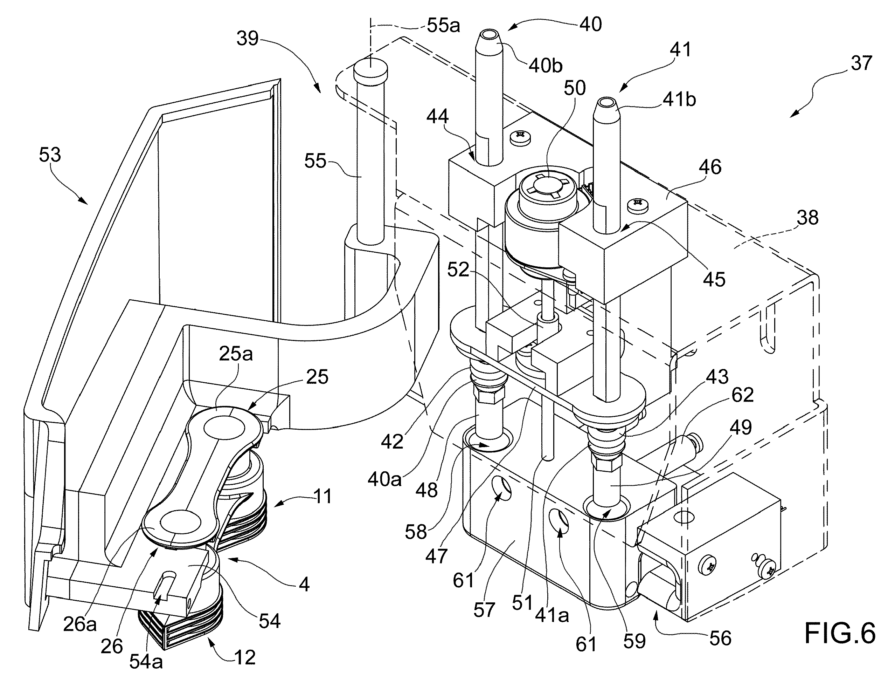

With reference to figure 6, the dialysis machine 37 comprises a frame 38 and a coupling assembly 39, which is mounted on the frame 38 so as to receive the connection 4 of the bag 1 and couple the connection 4 to a hydraulic circuit (not shown) of the machine 37, said circuit being suited to feed a solvent to the bag 1 and to draw, from the bag 1, a sodium bicarbonate solution obtained from the dissolution, inside the bag 1, of the bicarbonate powder in the solvent. For the sake of simplicity, figure 6 only shows the connection 4 of the bag 1.

The coupling assembly 39 comprises two ducts 40 and 41, each of which comprises a respective coupling end 40a, 41a, which is provided with at least one sealing ring 42, 43 so as to engage, in a tight and removable manner, the end portion 25, 26 of a respective passage element 11, 12 of the connection 4. The other ends 40b and 41b of the ducts are permanently connected to the hydraulic circuit. The ducts 40 and 41 can slide in two respective vertical circular guides 44 and 45, obtained in a sub-frame 46 fixed to the frame 38, so as to be able to vertically move between a raised waiting position, which is the one shown on figure 6, and a lowered operating position. The coupling ends 40a and 41a are rigidly joined by means of a horizontal bracket 47, so that the distance between the ducts 40 and 41 is equal to the distance between the passage elements 11 and 12 of the bag 1.

The two ducts 40 and 41 comprise two respective filters 48 and 49 so that, during the coupling to the respective passage elements 11 and 12, bicarbonate powder available inside the bag 1 is prevented from entering the hydraulic circuit. In particular, each filter 48, 49 is partially fitted into the coupling end 40a, 41a of the respective duct 40, 41 in such a way that a prevailing portion of the filter 48 and 49 projects from the coupling end 40a, 41a so as to be able to penetrate the respective passage element 11, 12, when the latter is engaged by the coupling end 40a, 41a of the relative duct 40, 41.

The coupling assembly 39 comprises a motor 50, which is mounted on the sub-frame 46 and whose drive shaft ends with a worm screw 51, and a threaded element 52, which is fixed to the bracket 47 and coupled to the worm screw 51 so as to allow the ducts 40, 41 to move between the raised position and the lowered position.

The coupling assembly 39 comprises, furthermore, a cover 53, which is hinged to the frame 38 and is provided with a support 54, which is suited to receive the connection 4 of the bag 1. The hinge 55 connecting the cover 53 to the frame 38 has a substantially vertical axis 55a. The support 54 consists of a U-shaped plate, which remains horizontal during the rotation of the cover 53 relative to the axis 55a and is suited to receive the end portions 25 and 26 of the passage elements 11 and 12 of the connection 4, so that the annular edges 25a and 26a rest against the edge of the plate. The cover 53 can be manually moved between an open position, which is the one shown in figure 6 and in which the bag 1 can be loaded by inserting the connection 4 into the support 54, and a closed position, in which the passage elements 11 and 12 are in position, coaxial with the respective ducts 40 and 41, and the panel of the cover 53 covers the ducts 40 and 41 on the outside. The coupling assembly 39 comprises a hooking means 56, which is suited to be coupled, in a releasable manner, to a recess 54a obtained in the support 54, so as to hold the cover 53 in the closed position.

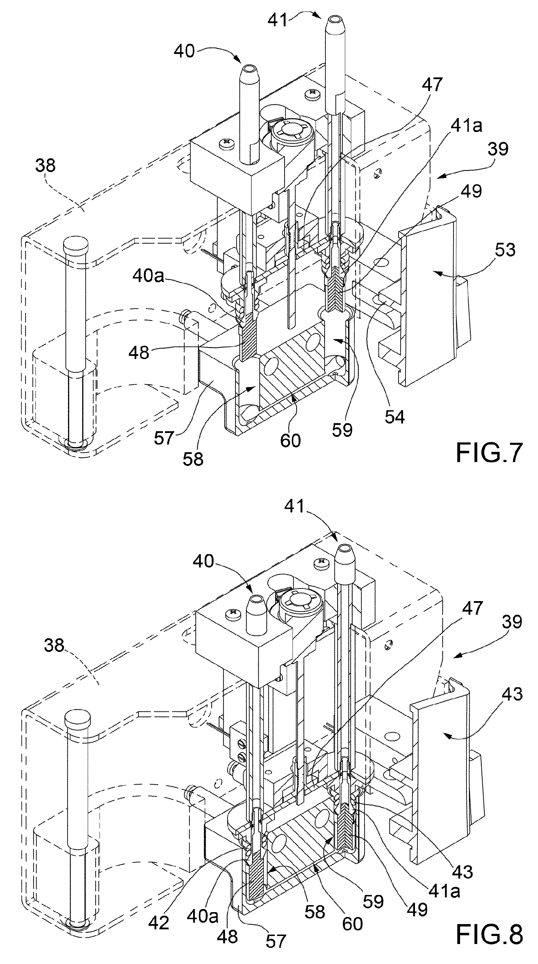

The dialysis machine 37 comprises a solid body 57, which has two vertical channels 58 and 59, having a distance that is equal to the one of the ducts 40 and 41, and has an inner duct 60 (figures 7 and 8), which establishes a mutual communication between the bottoms of the two channels 58 and 59. The body 57 has two holes 61, which are suited to be engaged, in a sliding manner, by two respective horizontal pins 62 fixed to the frame 38, so as to allow the body to horizontally move between an operating position, which is the one shown in figure 6 and in which each channel 58, 59 is coaxial to a respective duct 40, 41, and a retracted position, in which the body 57 leaves room for the connection 4. The body 57 is normally held in the operating position by an elastic element (not shown). The channels 58, 59 and 60 define a by-pass path, which is suited to close the hydraulic circuit of the dialysis machine 37, so as to allow the circuit itself to be washed.

Figure 7 shows, in a perspective view partially in section along the longitudinal axes of the ducts 40 and 41, the coupling assembly 39 in a by-pass path loading configuration, in which the channels 40 and 41 are in the raised waiting position, the body 57 is in the operating position, the bag is not inserted into the support 54, and the cover 53 is in the closed position. The U shape of the support 54, which is not completely visible in figure 7, has a width that is such as to embrace, with precision but without interference, the body 57, when the cover 53 is in the closed position.

Figure 8 shows, in the same view as figure 7, the coupling assembly 39 in a by-pass path sealing configuration, which differs from the configuration of figure 7 due to the fact that the ducts 40 and 41 are in the lowered operating position, with the coupling ends 40a and 41a engaging the respective channels 58 and 59, so that the filters 48 and 49 penetrate the channels 58 and 59 up to the level of the channel 60. The sealing rings 42 and 43 ensure a tight sealing between the outer surface of the coupling ends 40a and 41a and the inner surface of the channels 58 and 59. The washing of the hydraulic circuit is performed when the coupling assembly 39 is in the by-pass path sealing configuration.

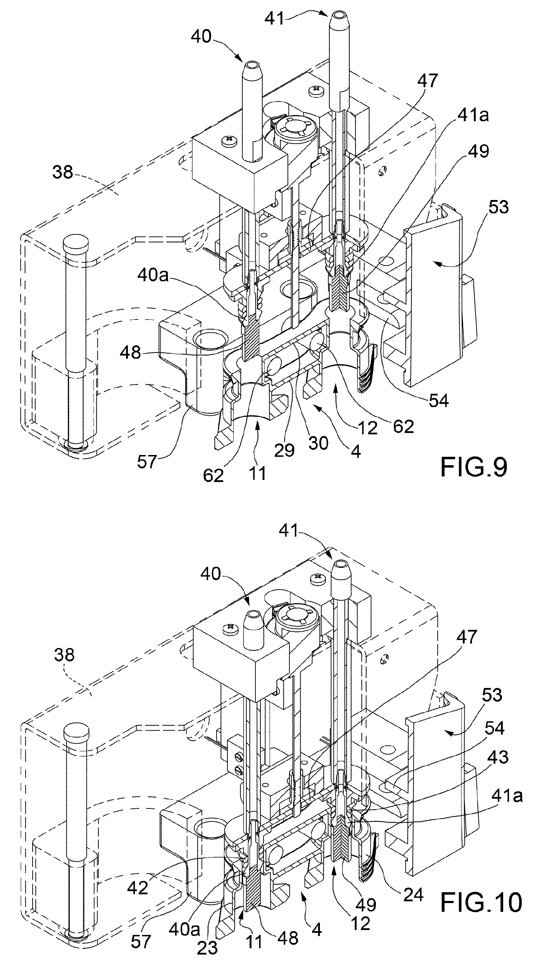

Figure 9 shows, in the same view as figure 7, the coupling assembly 39 in a bag 1 loading configuration, which differs from the configuration of figure 7 due to the fact that the connection 4 of the bag 1 is inserted into the support 54 and the cover 53, in the closed position, presses the connection 4 against the body 57 so as to hold the latter in the retracted position. The body 57, in the retracted position, leaves the pins 62 uncovered, which place themselves between the flat elements 29 and 30.

Figure 10 shows, in the same view as figure 7, the coupling assembly 39 in a bag 1 sealing configuration, which differs from the configuration of figure 9 due to the fact that the ducts 40 and 41 are in the lowered operating position, with the coupling ends 40a and 41a engaging the respective passage elements 11 and 12 of the connection 4, so that the filters 48 and 49 penetrate until they reach the inside of the base portions 23 and 24 of the passage elements 11 and 12. The sealing rings 42 and 43 ensure a tight sealing. When the coupling assembly 39 is in the bag 1 sealing configuration, the hydraulic circuit feeds the solvent to the bag 1 through, for example, the duct 40, which is tightly connected to the passage element 11, and the sodium bicarbonate solution is drawn from the bag 1 through the duct 41, which is tightly connected to the passage element 12.

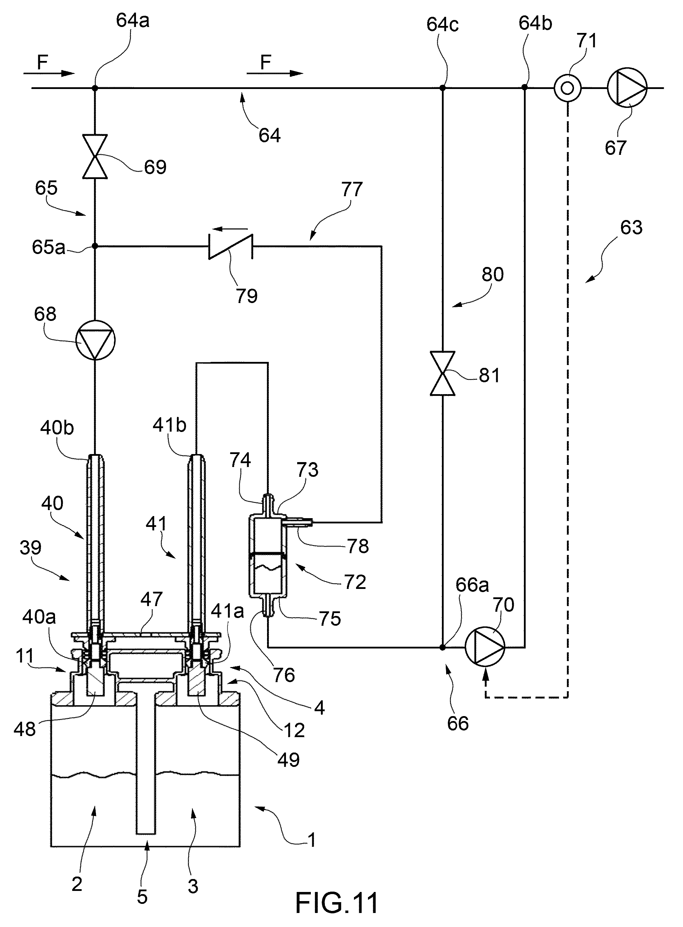

In figure 11, number 63 indicates, as a whole, the hydraulic circuit of the dialysis machine 37 for the distribution of a solvent and of a bicarbonate solution produced with said solvent. In particular, the circuit 63 is suited to feed a solvent to the bag 1 and to draw, from the bag 1, a saturated sodium bicarbonate solution produced, inside the bag 1, by the dissolution of the bicarbonate powder in the solvent. The circuit 63 comprises a main branch 64, which is flown through by the solvent according to a given circulation direction F, a solvent delivery branch 65, which connects a point 64a of the main branch 64 to the end 40b of the duct 40 of the coupling assembly 39 (figure 6), so as to the feed the solvent to the bag 1, and a solution return branch 66, which connects the end 41b of the duct 41 of the coupling assembly 39 to another point 64b of the main branch 64 arranged downstream of the point 64a relative to the circulation direction F, so as to receive the solution from the bag 1 and introduce the solution into the main branch 64. The outlet of the main branch 64, arranged downstream of the point 64b relative to the circulation direction F, supplies the bicarbonate solution to the dialysis filter (not shown).

The main body 64 comprises a pump 67, which is arranged downstream of the point 64b relative to the circulation direction F, so as to create a head that is such as to cause the solvent to circulate according to the direction F, oriented from the point 64a to the point 64 b.

The solvent delivery branch 65 comprises another pump 68 to push the solvent into the duct 40 and, thus, into the bag 1. Furthermore, the solvent delivery branch 65 comprises a pressure relief valve 69, which is arranged upstream of the pump 68 so as to limit the pressure of the solvent in the bag 1. The pressure in the bag 1 must be lower that 0.3 bar (30 kPa), otherwise there is a risk of explosion.

The solution return branch 66 comprises a pump 70 to push the solution towards the main branch 64 during the steady-state operation of the dialysis machine 37. The bicarbonate solution that reaches the solution return branch 66 mixes with the solvent coming from point 4a in the point 64b of the main branch 64. Therefore, the bicarbonate percentage dissolved in the solution at the outlet of the main branch 64, namely downstream of the point 64b relative to the circulation direction F, is linked to the bicarbonate percentage of the solution in the solution return branch 66.

The main branch 64 comprises, downstream of the point 64b relative to the circulation direction F, and in particular between the point 64b and the inlet of the pump 67, conductivity sensor 71 to provide a signal indicating the conductivity of the solution at the outlet of the main branch 64. The conductivity of the solution is liked to the percentage of bicarbonate dissolved. The pump 70 is controlled as a function of the signal provided by the conductivity sensor 71, so as to keep the percentage of bicarbonate downstream of the point 64b at a desired value.

The solvent return branch 66 comprises a dripper 72 to prevent the gases generated with the production of the solution to be introduced into the main branch 64. The gases generated during the production of the solution comprise carbon dioxide, which is a product of the reaction between the solvent and the bicarbonate powder, and air in the form of bubbles, which is released by the breaking of the particles of the bicarbonate powder when the latter is dissolved in the solvent.

The dripper 72 is arranged between the duct 41 and the pump 70. In particular, the dripper 72 consists of a container having a preferably - but not necessarily - cylindrical shape, whose ceiling 73 has an inlet 74 that is connected to the end 41b of the duct 41 and whose bottom 75 has an outlet 76 that is connected to the inlet of the pump 70. The dimensions of the dripper 72 are chosen as a function of the capacity of the bag 1. During the steady-state operation, the dripper 72 must remain substantially full of solution, so as to compensate possible emptying events caused by the air bubbles coming from the bag 1. Advantageously, the dripper 72 has a capacity ranging from 5 to 20 cm³. Higher capacity values are not recommended, because they would excessively increase the washing times of the circuit 63.

The circuit 63 comprises a gas recirculation branch 77, which connects a further outlet 78 of the dripper 72, obtained in the ceiling 73 of the dripper 72, and a point 65a of the solvent delivery branch 65, arranged between the pump 68 and the pressure relief valve 69, so as to introduce the gases generated with the production of the solution into the solvent delivery branch 65. The gas recirculation branch 77 comprises a non-return valve 79 to prevent the solvent circulating in the solvent delivery branch 65 to directly flow into the solution return branch 66 without flowing through the bag 1. The gas recirculation branch 77 brings the gases gathered in the dripper 72 back into the bag 1, so as to prevent the gases themselves from flowing through the pumps 70 and 67 and entering the main branch 64, whose outlet is connected to the dialysis filter.

The circuit 63 comprises, furthermore, a further solution return branch 80, which connects a point 66a of the other solution return branch 66 to a point 64c of the main branch 64 arranged upstream of the point 64b and comprises an on/off valve 81, which is controlled by the control unit (not shown) of the dialysis machine 37 so as to define a by-pass path for the solution that allows the initial filling and the final emptying of the bag 1 to be quickened. In particular, the point 66a is arranged between the outlet 76 of the dripper 77 and the inlet of the pump 70 and the point 64c is arranged between the point 64a and the point 64b.

In use, the on/off valve 81 is opened for an initial period so as to allow both pockets 2 and 3 of the bag 1 to be quickly filled with the solvent. A the end of the initial period, namely when the pockets 2 and 3 are presumably full of solvent, a steady-state operating period starts, during which the on/off valve 81 is closed so as to cause the solution to circulate only in the solution return branch 66, so that the flow solution directed at the main branch 64 is regulated by the pump 70. A the end of the steady-state operating period, namely when the bicarbonate of the bag 1 is close to being completely used up, a final period starts, during which the on/off valve 81 is reopened so as to allow the bag 1 to be quickly emptied.

According to a further embodiment of the circuit 63, which is not shown, the pump 67 is arranged upstream of the conductivity sensor 71, in particular between the point 64b and the conductivity sensor 71.

According to a further embodiment of the circuit 63, which is not shown, the solvent delivery branch 65 comprises an adjusted narrowing, which is arranged between the point 65a and the inlet of the pump 68, so as create a vacuum upstream of the pump 68 that is such as to draw gas or gas mixed with solution from the dripper 72.

One of the advantages of the dialysis machine 37 is that it permits the use of sodium bicarbonate bags without filters, such as bag 1, since the dialysis machine 37 already mounts the filters 48 and 49 for the bicarbonate powder on board the coupling assembly 39. Another advantage is that the gases generated with the production of sodium bicarbonate are prevented from flowing into the main branch 64 of the circuit 63, thanks to the presence of the dripper 72 on the solution return branch 66. Another advantage is a higher speed in the bag 1 filling and emptying steps, thanks to the presence of the further solvent return branch 80 provided with the on/off valve 81.

## Claims

1. A bag containing bicarbonate powder, the bag (1) being divided into two pockets (2, 3), which are arranged one next to the other, have respective bottom portions (6, 7) and contain bicarbonate powder (B), comprising a lower channel (5), which establishes a communication between said bottom portions (6, 7), and the bag lacking any kind of dip tube and/or filter and being **characterized in that** the lower channel is U-shaped and has a central section (8) that is arranged under the minimum level of both bottom portions (6, 7), so that the lower channel (5), in use, is normally full of bicarbonate powder.

2. A bag according to claim 1, wherein said two pockets (2, 3) are symmetrical relative to a longitudinal axis (13a) of the bag (1) and said bottom portions (6, 7) have respective lower openings (15, 16), which are arranged in symmetrical positions relative to the longitudinal axis (13a); said lower channel (5) being symmetrical relative to the longitudinal axis (13a) and establishing a communication between said lower openings (15, 16).

3. A bag according to claim 2, wherein said lower openings (15, 16) are arranged is close positions relative to said longitudinal axis (13a) and said bottom portions (6, 7) are inclined towards the respective lower openings (15, 16) in a specular manner relative to said longitudinal axis (13a).

4. A bag according to claim 3, wherein each one of said bottom portions (6, 7) comprises a respective portion (6a, 7a) joined to the respective lower opening (15, 16) and inclined according to an angle (α), relative to a direction that is perpendicular to said longitudinal axis (13a), ranging from 5° to 15°.

5. A bag according to any of the claims from 2 to 4, wherein said two pockets (2, 3) have a same first width (LP), which is measured perpendicular to the longitudinal axis (13a); each one of said lower openings (15, 16) having a second width (LB), which is measured perpendicular to the longitudinal axis (13a) in a flattening plane of the empty bag (1) and is smaller than the half of said first width (LP).

6. A bag according to any of the claims from 2 to 5, wherein each one of said lower openings (15, 16) has a second width (LB), which is measured perpendicular to the longitudinal axis (13a) in a flattening plane of the empty bag (1), and each branch of said lower channel (5) has a first length (H20), which is measured parallel to said longitudinal axis (13a) and is greater than said second width (LB).

7. A bag according to any of the claims from 1 to 6, wherein each branch of said lower channel (5) has a first length (H20), which is measured parallel to said longitudinal axis (13a), and said central section (8) of the lower channel (5) has a depth (H8), which is measured along said longitudinal axis (13a) and is smaller than two thirds of said first length (H20).

8. A bag according to any of the claims from 1 to 7, wherein said lower channel (5) comprises two branches, each of which has a first length (H20), which is measured parallel to said longitudinal axis (13a), an inner side (14) and an outer side (20), the latter comprising a central straight segment (20a) having a second width (L20), which is measured parallel to said longitudinal axis (13a) and is approximately equal to said first length (H20).

9. A bag according to any of the claims from 1 to 8, wherein said pockets (2, 3) and said lower channel (5) are manufactured as one single piece from a plastic film that is folded in two parts and welded.

10. A bag according to claim 9, wherein said lower channel (15) comprises an inner side (14) and an outer side (20); the bag having a central welded joint (13) extending along said longitudinal axis (13a) so as to divide the bag (1) into said two pockets (2, 3) and define said inner side (14).

11. A bag according to claim 10, wherein said bottom portions (6, 7) have respective lower openings (15, 16), which are arranged on opposite side of and contiguous with said central welded joint (13).

12. A bag according to any of the claims from 1 to 11, wherein said two pockets (2, 3) have respective upper openings (9, 10).

13. A bag according to claim 12 and comprising an upper connection (4) for the connection to a dialysis machine (37), the upper connection (4) comprising two passage elements (11, 12), rigidly connected to one another, each of which is welded in a tight manner to the upper opening (9, 10) of a respective pocket (2, 3), so as to allow a solvent to be introduced into the bag (1) through a first passage element (11) and a saturated bicarbonate solution to flow out of the bag (1) through the other passage element (12).

14. A bag according to claim 13, wherein said upper openings (9, 10) are arranged in a symmetrical and close position relative to a longitudinal axis (13a) of the bag (1).

15. A bag according to 12 or 13, wherein said two pockets (2, 3) have a same first width (LP), which is measured perpendicular to a longitudinal axis (13a) of the bag (1); each one of said upper openings (9, 10) having a third width (LS), which is measured perpendicular to the longitudinal axis (13a) of the bag (1) and is equal to approximately one third of said first width (LP).

## Patentansprüche

1. Ein Beutel der Bicarbonatpulver enthält, wobei der Beutel (1) in zwei Taschen (2, 3) unterteilt ist, die nebeneinander angeordnet sind und entsprechende Bodenteile (6, 7) aufweisen und Bicarbonatpulver (B) enthalten, wobei ein unterer Kanal (5) vorgesehen ist, der eine Verbindung zwischen den erwähnten Bodenteilen (6, 7) herstellt, und wobei der Beutel keinerlei Tropfrohr und/oder Filter aufweist und **dadurch gekennzeichnet ist, dass** der untere Kanal U-förmig ist und einen Mittelabschnitt (8) aufweist, der unter dem Minimalniveau der Bodenteile (6, 7) derart angeordnet ist, dass der untere Kanal (5) im Gebrauch, normalerweise mit Bicarbonatpulver gefüllt ist.

2. Ein Beutel nach Anspruch (1), wobei die zwei Taschen (2, 3) symmetrisch bezüglich einer Längsachse (13a) des Beutels (1) sind und wobei die Bodenteile (6, 7) entsprechende untere Öffnungen (15, 16) aufweisen, die in symmetrischen Positionen relativ zu der Längsachse (13a) angeordnet sind; wobei ferner der untere Kanal (5) symmetrisch bezüglich der Längsachse (13a) ist und eine Verbindung zwischen den unteren Öffnungen (15, 16) herstellt.

3. Ein Beutel nach Anspruch 2, wobei die unteren Öffnungen (15, 16) in nahen Positionen bezüglich der Längsachse (13a) angeordnet sind und die erwähnten Bodenteile (6, 7) zu den entsprechenden unteren Öffnungen (15, 16) hin geneigt sind in einer gespiegelten Art und Weise relativ zu der erwähnten Längsachse (13a).

4. Ein Beutel nach Anspruch (3), wobei jeder einzelne der erwähnten Bodenteile (6, 7) einen entsprechenden Teil (6a, 7a) aufweist verbunden mit der entsprechenden unteren Öffnung (15, 16) und geneigt entsprechend einem Winkel α relativ zu einer Richtung die senkrecht zu der in Längsachse (13a) verläuft, wobei der Winkel im Bereich von 5° bis 15° liegt.

5. Ein Beutel nach einem der Ansprüche 2 bis 4, wobei die erwähnten zwei Taschen (2, 3) die gleiche erste Breite (LP) gemessen senkrecht zu der Längsachse (13a) besitzen; wobei jede der erwähnten unteren Öffnungen (15, 16) eine zweite Breite (LB) besitzt, die gemessen ist senkrecht zur Längsachse (13a) in einer abgeflachten Ebene des Beutels (1) und die kleiner ist als die Hälfte der ersten Breite (LP).

6. Ein Beutel nach einem der Ansprüche 2 bis 5, wobei jede der erwähnten unteren Öffnungen (15, 16) eine zweite Breite (LB) besitzt, die senkrecht zur Längsachse (13a) in einer abgeflachten Ebene des leeren Beutels gemessen ist; wobei jeder Zweig des erwähnten unteren Kanals (5) eine erste Länge (H20) besitzt, die gemessen ist parallel zu der Längsachse (13a) und die größer ist als die zweite Breite (LB).

7. Ein Beutel nach einem der Ansprüche 1 bis 6, wobei jeder Zweig des erwähnten unteren Kanals (5) eine erste Länge (H20) besitzt die parallel zu der erwähnten Längsachse (13a) gemessen ist und wobei der erwähnte mittlere Abschnitt (8) des erwähnten unteren Kanals (5) eine Tiefe (H8) besitzt, die längs der Längsachse (13a) gemessen ist und die kleiner ist als zwei Drittel der erwähnten ersten Länge (H20).

8. Ein Beutel nach einem der Ansprüche 1 bis 7, wobei der untere Kanal (5) zwei Zweige aufweist deren jeder eine erste Länge (H20) besitzt, die gemessen ist parallel zu der Längsachse (13a) und mit einer Innenseite (14) und einer Außenseite (20), wobei Letztere ein mittiges, geradliniges Segment (20a) aufweist mit einer zweiten Breite (L20) welche gemessen ist parallel zu der erwähnten Längsachse (13a) und annähernd gleich ist der ersten Länge (H20).

9. Ein Beutel nach einem der Ansprüche 1 bis 8, wobei die erwähnten Taschen (2, 3) und der erwähnte untere Kanal (5) als ein einziges Teil aus einem Kunststofffilm bzw. -schicht geformt sind und zwar gefaltet in zwei Teile und verschweißt.

10. Ein Beutel nach Anspruch 9, wobei der untere Kanal (15) eine Innenseite (15) und eine Außenseite (20) aufweist, wobei der Beutel eine mittlere geschweißte Verbindung (13) besitzt, die sich entlang der Längsachse (13a) erstrecht, um so den Beutel (1) in zweit Taschen (2, 3) aufzuteilen und die erwähnte Innenseite (14) zu definieren.

11. Ein Beutel nach Anspruch 10, wobei die erwähnten Bodenteile (6, 7) entsprechende untere Öffnungen (15, 16) aufweisen, die auf der entgegensetzten Seite der mittigen geschweißten Verbindung (13) und angrenzend daran angeordnet sind.

12. Ein Beutel nach einem der Ansprüche 1 bis 11, wobei die erwähnten zwei Taschen (2, 3) entsprechende obere Öffnungen (9, 10) aufweisen.

13. Ein Beutel nach Anspruch 12 mit einer oberen Verbindung (4) für die Verbindung mit einer Dialysemaschine (37), wobei die obere Verbindung (4) zwei Durchlasselemente (11, 12) aufweist und zwar starr verbunden miteinander, wobei jedes in einer dichten Art und Weise mit der oberen Öffnung (9, 10) in einer entsprechenden Tasche (2, 3) verschweißt ist um so zu gestatten, dass ein Lösungsmittel in dem Beutel (1) durch ein erstes Durchlasselement (11) eingeführt werden kann und dass eine gesättigte Bicarbonatlösung aus dem Beutel (1) durch das andere Durchlasselement (12) laufen kann.

14. Ein Beutel nach Anspruch 13, wobei die oberen Öffnungen (9, 10) in einer symmetrischen und dichten Position relativ zu der Längsachse (13a) des Beutels (1) angeordnet sind.

15. Ein Beutel nach Anspruch 12 oder 13, wobei die erwähnten zwei Taschen (2, 3) eine gleiche erste Breite (LP) besitzen, die senkrecht zu einer Längsachse (13a) des Beutels gemessen sind; wobei jeder der erwähnten oberen Öffnungen (9, 10) eine dritte Breite (LS) besitzt, die gemessen ist senkrecht zu der Längsachse (13a) des Beutels (1) und die annähernd gleich ist einem Drittel der erwähnten ersten Breite (LP).

## Revendications

1. Sachet contenant du bicarbonate en poudre, le sachet (1) étant divisé en deux poches (2, 3), qui sont agencées l'une à côté de l'autre, comportent des parties inférieures respectives (6, 7) et renferment du bicarbonate en poudre (B), comprenant un canal inférieur (5), qui crée une communication entre lesdites parties inférieures (6, 7), et le sachet étant dépourvu de tout type de tube plongeur et/ou filtre et étant **caractérisé en ce que** le canal inférieur a la forme d'un U et comporte une section centrale (8) qui est agencée sous le niveau minimum des deux parties inférieures (6, 7), de manière que le canal inférieur (5), en service, soit normalement plein de bicarbonate en poudre.

2. Sachet selon la revendication 1, dans lequel lesdites deux poches (2, 3) sont symétriques relativement à un axe longitudinal (13a) du sachet (1) et lesdites parties inférieures (6, 7) comportent des ouvertures inférieures (15, 16) respectives, qui sont agencées en des positions symétriques relativement à l'axe longitudinal (13a) ; ledit canal inférieur (5) étant symétrique relativement à l'axe longitudinal (13a) et créant une communication entre lesdites ouvertures inférieures (15, 16).

3. Sachet selon la revendication 2, dans lequel lesdites ouvertures inférieures (15, 16) sont agencées en des positions proches relativement audit axe longitudinal (13a) et lesdites parties inférieures (6, 7) sont inclinées vers les ouvertures inférieures (15, 16) respectives de manière spéculaire relativement audit axe longitudinal (13a).

4. Sachet selon la revendication 3, dans lequel chacune desdites parties inférieures (6, 7) comprend une partie respective (6a, 7a) raccordée à l'ouverture inférieure respective (15, 16) et inclinée suivant un angle (α), relativement à une direction qui est perpendiculaire audit axe longitudinal (13a), de 5° à 15°.

5. Sachet selon l'une quelconque des revendications 2 à 4, dans lequel lesdites deux poches (2, 3) ont une même meilleure première largeur (LP), qui est mesurée perpendiculairement à l'axe longitudinal (13a) ; chacune desdites ouvertures inférieures (15, 16) ayant une deuxième largeur (LB), qui est mesurée perpendiculairement à l'axe longitudinal (13a) dans un plan d'aplatissement du sachet vide (1) et est inférieure à la moitié de ladite première largeur (LP).

6. Sachet selon l'une quelconque des revendications 2 à 5, dans lequel chacune desdites ouvertures inférieures (15, 16) a une deuxième largeur (LB), qui est mesurée perpendiculairement à l'axe longitudinal (13a) dans un plan d'aplatissement du sachet vide (1), et chaque ramification dudit canal inférieur (5) a une première longueur (H20), qui est mesurée parallèlement audit axe longitudinal (13a) et est supérieure à ladite deuxième largeur (LB).

7. Sachet selon l'une quelconque des revendications 1 à 6, dans lequel chaque ramification dudit canal inférieur (5) a une première longueur (H20), qui est mesurée parallèlement audit axe longitudinal (13a), et ladite section centrale (8) du canal inférieur (5) a une profondeur (H8), qui est mesurée le long dudit axe longitudinal (13a) et est inférieure aux deux tiers de ladite première longueur (H20).

8. Sachet selon l'une quelconque des revendications 1 à 7, dans lequel ledit canal inférieur (5) comprend deux ramifications, chacune ayant une première longueur (H20), qui est mesurée parallèlement audit axe longitudinal (13a), un côté intérieur (14) et un côté extérieur (20), ce dernier comprenant un segment droit central (20a) ayant une deuxième largeur (L20), qui est mesurée parallèlement audit axe longitudinal (13a) et est approximativement égale à ladite première longueur (H20).

9. Sachet selon l'une quelconque des revendications 1 à 8, dans lequel lesdites poches (2, 3) et ledit canal inférieur (5) sont fabriqués d'une seule pièce à partir d'un film plastique qui est plié en deux parties et soudé.

10. Sachet selon la revendication 9, dans lequel ledit canal inférieur (5) comprend un côté intérieur (14) et un côté extérieur (20) ; le sachet comportant un joint soudé central (13) s'étendant le long dudit axe longitudinal (13a) afin de diviser le sachet (1) en lesdites deux poches (2, 3) et de délimiter ledit côté intérieur (14).

11. Sachet selon la revendication 10, dans lequel lesdites parties inférieures (6, 7) comportent des ouvertures inférieures (15, 16) respectives, qui sont agencées sur le côté opposé de, et contiguës au, joint soudé central (13).

12. Sachet selon l'une quelconque des revendications 1 à 11, dans lequel lesdites deux poches (2, 3) comportent des ouvertures supérieures (9, 10) respectives.

13. Sachet selon la revendication 12 et comprenant une connexion supérieure (4) pour la connexion à une machine de dialyse (37), la connexion supérieure (4) comprenant deux éléments formant passages (11, 12), raccordés rigidement l'un à l'autre, chacun étant soudé de manière étanche à l'ouverture supérieure (9, 10) d'une poche (2, 3) respective, afin de permettre à un solvant d'être introduit dans le sachet (1) par un premier élément formant passage (11) et une solution de bicarbonate saturée de s'écouler du sachet (1) par l'autre élément formant passage (12).

14. Sachet selon la revendication 13, dans lequel lesdites ouvertures supérieures (9, 10) sont agencées de façon symétrique et à proximité d'un axe longitudinal (13a) du sachet (1).

15. Sachet selon la revendication 12 ou 13, dans lequel lesdites deux poches (2, 3) ont une première largeur (LP), qui est mesurée perpendiculairement à un axe longitudinal (13a) du sachet (1) ; chacune desdites ouvertures supérieures (9, 10) ayant une troisième largeur (LS), qui est mesurée perpendiculairement à l'axe longitudinal (13a) du sachet (1) et est égale à approximativement un tiers de ladite première largeur (LP).
